(19) Europäisches Patentamt / European Patent Office / Office européen des brevets

(11) **EP 2 440 189 B1**

(12) **FASCICULE DE BREVET EUROPEEN**

(45) Date de publication et mention de la délivrance du brevet:
**21.09.2016 Bulletin 2016/38**

(21) Numéro de dépôt: **10725419.5**

(22) Date de dépôt: **14.06.2010**

(51) Int Cl.:
*A61K 9/20* (2006.01)          *A61K 9/28* (2006.01)
*A61K 31/485* (2006.01)          *A61P 25/04* (2006.01)

(86) Numéro de dépôt international:
**PCT/EP2010/058323**

(87) Numéro de publication internationale:
**WO 2010/142814 (16.12.2010 Gazette 2010/50)**

(54) **RÉDUCTION DES FLUCTUATIONS PLASMATIQUES D'OPIOIDES**

MINIMIERUNG VON KONZENTRATIONSSCHWANKUNGEN EINES OPIOIDS IM BLUT

REDUCTION OF OPIOID BLOOD FLUCTUATIONS

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **12.06.2009 FR 0953951**
**12.06.2009 US 213483 P**

(43) Date de publication de la demande:
**18.04.2012 Bulletin 2012/16**

(73) Titulaire: **ETHYPHARM**
**92210 Saint-Cloud (FR)**

(72) Inventeurs:
• **HERRY, Catherine**
**F-27670 Saint-Ouen du Tilleul (FR)**
• **BOYER, Maryline**
**F-78430 Louveciennes (FR)**
• **VAUZELLE-KERVROEDAN, Françoise**
**F-91470 Les Molières (FR)**
• **OURY, Pascal**
**F-78150 Le Chesnay (FR)**

(74) Mandataire: **Regimbeau**
**20, rue de Chazelles**
**75847 Paris Cedex 17 (FR)**

(56) Documents cités:
**WO-A1-2007/099152     WO-A1-2007/099154**

Remarques:
Le dossier contient des informations techniques présentées postérieurement au dépôt de la demande et ne figurant pas dans le présent fascicule.

**Description**

La présente invention a pour objet un comprimé matriciel à libération prolongée d'au moins un opioïde pour son utilisation en tant que médicament permettant de diminuer les fluctuations de concentrations plasmatiques dudit opioïde.

Il est bien connu que lorsque les opioïdes sont administrés par voie orale, ils ne sont efficaces que s'ils sont donnés assez fréquemment et en doses suffisamment élevées.

L'activité des opioïdes chez l'homme est directement liée à la concentration plasmatique d'opioïdes. De plus, il y a un rapport général entre la concentration croissante plasmatique d'opioïdes et la fréquence croissante des effets secondaires défavorables liés à la dose, en particulier les nausées, les vomissements, la somnolence et la dépression respiratoire.

Ainsi, le traitement et la gestion de douleur par l'utilisation de formes galéniques contenant des principes actifs tels que les opioïdes, nécessitent que la libération dudit principe actif se fasse sur des périodes prolongées de manière à fournir un effet thérapeutique efficace. Il est également souhaitable que les formulations pharmaceutiques comprenant des opioïdes fournissent une dose suffisante tout en évitant les variations importantes de concentrations plasmatiques (peak to trough) et les épisodes de résurgence de douleur ou de douleurs aigües (breakthrough pain) chez les patients.

Parmi les opioïdes forts, la morphine est probablement la molécule de prédilection pour le traitement des douleurs sévères. La morphine est bien absorbée par voie orale, mais subit une forte extraction hépatique, de sorte que sa biodisponibilité peut diminuer jusqu'à 20-30%.

Il existe plusieurs formes pharmaceutiques de morphine commercialisées par exemple sous les noms de Skenan® et Moscontin®. Ces formulations pharmaceutiques ont une durée d'action de 12 heures, et sont dosées à 10, 30, 60, 100 mg et 200 mg de sulfate de morphine. Ces formes pharmaceutiques doivent être administrées au moins deux fois par jour.

Le Kapanol®, présenté sous forme de granules dans des capsules dosées à 20, 50 et 100 mg, a une durée d'action de 24 heures.

Il existe d'autres opioïdes forts tels que l' hydromorphone ou l'oxycodone.

L'hydromorphone (Sophidone® dosé à 4, 8, 16 et 24 mg) est un dérivé semi-synthétique de la morphine dont la puissance analgésique est de l'ordre de 7,5 fois celle de la morphine. C'est une alternative orale à la morphine. Elle est indiquée lors de douleurs intenses d'origine cancéreuse, en cas de résistance et/ou d'intolérance (exemples : troubles cognitifs, somnolence et hallucinations) ou de résistance à la morphine.

L'oxycodone est un dérivé semi-synthétique de la morphine dont la puissance analgésique est de l'ordre de deux fois celle de la morphine. C'est une alternative orale à la morphine pour différentes raisons pharmacologiques, notamment en cas de résistance ou d'intolérance.

Elle est commercialisée sous une forme à libération prolongée (Oxycontin LP® ou Oxygesic® 10, 20 et 40, 80 mg) et immédiate (Oxynorm® 5, 10, 20 mg). L'indication de l'autorisation de mise sur le marché (AMM) la réserve au traitement des douleurs intenses d'origine cancéreuse ou en cas de résistance ou d'intolérance à la morphine.

On peut également citer le document WO2004084868 qui décrit une forme pharmaceutique à libération prolongée comprenant une matrice à base d'un polyéthylène glycol, d'un polyéthylène oxyde et d'un bloc copolymère d'éthylène oxyde incluant un opioïde et un enrobage à base de dérivés cellulosiques qui libère plus de 75 % de l'opioïde entre 4 et 10 heures selon une cinétique d'ordre zéro. Ce document enseigne également que les compositions commerciales MS Contin® (administrée deux fois par jour) et Kadian® (administrée une fois par jour) montrent des fluctuations de concentrations plasmatiques telles que le minimum de concentration plasmatique est inférieur à la moitié de la concentration plasmatique maximale.

Le document EP0609961 décrit une composition pharmaceutique à libération prolongée comprenant un noyau contenant un agoniste opiacé et un enrobage contenant un polymère insoluble indépendamment du pH, un composé soluble dans un acide à un pH de 1 à 4 et un polymère entérosoluble, à peu près insoluble à un pH de 1 à 4, ladite composition libérant l'opiacé, à l'état stationnaire avec un $T_{max}$ de 4,5 heures ou plus.

Le document WO 96/00066 décrit des comprimés de morphine, pour administration orale, à libération contrôlée. Ces comprimés sont obtenus par compression successive de deux compositions : une première composition dite "thérapeutique" obtenue par granulation humide d'un mélange contenant du sulfate de morphine, un ou plusieurs polyoxydes d'alkylène et de la polyvinylpyrrolidone, et une deuxième composition obtenue par granulation humide d'un mélange contenant un polyoxyde d'alkylène, du chlorure de sodium et de l'hydroxypropylméthylcellulose. Le comprimé ainsi obtenu comprend une composition de principe actif enrobée d'une membrane semi-perméable poreuse qui contrôle la vitesse de libération dudit principe actif.

Les brevets US4,861,598, et US4,970,075 décrivent des compositions pharmaceutiques à libération prolongée pouvant inclure l'oxycodone. Les compositions pharmaceutiques décrites contiennent comme excipients un alcool aliphatique contenant de 10-18 atomes de carbone et une résine acrylique. Dans une composition exemplifiée, 43% d'oxycodone est libéré en une heure et 100% en 5 heures ; dans un autre exemple, 16% d'oxycodone est libéré en une heure et 100% en 9 heures.

Les demandes WO2007/099154 et WO2007/0099152 déposées par la Demanderesse, décrivent des comprimés matriciels insolubles dans l'eau capables de libérer de l'oxycodone dans l'organisme de façon prolongée dans le temps, préférentiellement pendant des périodes supérieures à 12 heures et plus préférentiellement supérieures à 20 heures.

Les comprimés matriciels enrobés d'éthylcellulose également appelés comprimés « QD » qui sont décrits dans les demandes WO2007/099154, et WO2007/099152 ont été développés pour être administrés en une seule prise par jour (forme once-a-day).

Un objectif essentiel de la présente invention est donc d'améliorer la gestion de la douleur par l'utilisation de formulations pharmaceutiques d'opioïde à libération prolongée présentant des performances pharmacocinétiques telles que les fluctuations de concentrations plasmatiques d'opioïdes sont fortement réduites.

DEFINITIONS

On parle de comprimé matriciel selon l'invention pour désigner un comprimé dont la structure interne est homogène et identique du centre vers la périphérie du comprimé. Ainsi, les comprimés de la présente invention, sont composés d'un mélange homogène de principe actif sous forme de poudre ou de granules et d'une matrice de compression à base d'au moins un excipient choisi dans le groupe comprenant les polymères retard pH indépendants et insolubles dans l'eau, les excipients minéraux et leurs mélanges, tel que défini dans la revendication 1.

Dans le cadre de la présente invention, on parlera de matrice de compression pour désigner l'ensemble des excipients qui participent à la cohésion du comprimé. Ladite matrice de compression est à la fois insoluble dans l'eau, et possède une certaine perméabilité (matrice hydrophile) ou un réseau poreux (matrice inerte) responsable de la libération progressive de l'actif, qui ne varie pas en fonction des conditions de pH du milieu.

Le terme " mélange pour compression " est employé dans la présente demande pour désigner l'ensemble des constituants du comprimé (le ou les principes actifs, granulés ou non et les constituants de la matrice de compression) avant sa compression sous forme de comprimé.

Au sens de la présente invention, l'expression " état d'équilibre (steady-state) ", signifie l'état pharmacocinétique stationnaire qui est atteint après des prises répétées de la même forme galénique. Cet état d'équilibre est généralement atteint après 4 à 5 demi-vies du médicament avec des oscillations de concentrations plasmatiques de l'opioïde entre un $Css_{max}$ et un $Css_{min}$.

L'évaluation en prises répétées permet de mesurer les paramètres habituels de biodisponibilité (AUCss, $Css_{max}$, $Tss_{max}$), mais rend compte également de l'importance des fluctuations entre les concentrations maximales ($Css_{max}$) et minimales ($Css_{min}$) à l'équilibre.

$C_{max}$ ou concentration plasmatique maximale est le pic de concentration représentant le point où la concentration plasmatique est la plus élevée de toute la cinétique. Ce pic est mesuré après une prise d'une dose unique d'un opioïde.

$Css_{max}$ signifie la concentration plasmatique maximale à l'état déquilibre.

$T_{max}$ est la valeur du temps nécessaire pour atteindre la concentration plasmatique maximale. Cette valeur est indicative de la vitesse d'absorption d'une substance pharmaceutiquement active.

$Tss_{max}$ signifie la valeur du temps nécessaire pour atteindre la concentration plasmatique maximale à l'état d'équilibre.

Les paramètres pharmacocinétiques $C_{max}$ et $T_{max}$ sont directement déduits des points expérimentaux.

L'administration répétée d'un médicament peut impliquer une certaine accumulation du médicament, ou de ses métabolites, dont l'importance dépend du schéma posologique utilisé.

Soit un médicament M, administré par voie orale à une dose D avec un intervalle de temps d'administration $\tau$. Le médicament est donné alors que la dose précédente n'est pas encore totalement éliminée, la quantité administrée s'ajoute donc à la quantité non éliminée provenant des doses précédentes (principe de la superposition). Après un certain temps, la quantité de médicament absorbé entre dans un certain équilibre de vitesse avec la quantité de médicament éliminé pendant l'intervalle $\tau$ ; un état d'équilibre est alors atteint. Toute réadministration de produit à intervalle $\tau$ ne modifiera plus cet état. Les concentrations fluctuent entre une concentration minimale $Css_{min}$ et une concentration maximale $Css_{max}$. Lorsque la vitesse d'entrée est parfaitement équilibrée avec la vitesse d'élimination, cet état d'équilibre est représenté par un pseudo-plateau, $Css_{max}$ et $Css_{min}$ sont alors très proches.

Au sens de la présente invention, on entend par «administration orale répétée», l'administration de la formulation de la présente invention à une dose D avec un intervalle de temps d'administration $\tau$ qui est compris entre 8 et 14 heures, de préférence compris entre 11 et 13 heures, de manière particulièrement préférée qui est de 12 heures.

Les paramètres «fluctuation» et «swing» sont exprimés en % et sont calculés selon les équations suivantes:

$$\text{-Fluctuation} : 100^* (C_{max} - C_{min})/Cav$$

Avec Cav, la concentration moyenne («concentration average»), représentant l'AUC (aire sous la courbe) sur l'intervalle de temps divisé par l'intervalle de temps: $Cav = AUC\tau/\tau$

Au sens de la présente invention, l'expression « réduction de fluctuations » peut signifier la réduction du nombre de pics et vallées ou de préférence une valeur du paramètre « fluctuation » compris entre 25% et 50%, ou encore plus de préférence inférieur à 25%.

$$\text{-Swing} : 100^*(C_{max} - C_{min})/C_{min} = \Delta$$

On entend par un profil plasmatique montrant des « pics et vallées » un profil plasmatique avec des modulations très prononcées, le pic pouvant correspondre au $C_{max}$ et la vallée à une concentration plasmatique obtenue après élimination du principe actif.

Il est donc très avantageux de disposer d'une forme à libération modifiée pouvant permette d'obtenir un profil de concentration plasmatique en plateau que l'on obtiendrait après perfusion à débit constant afin de niveler les phénomènes de pics et de vallées.

Le temps de demi-vie T½ est l'intervalle de temps nécessaire pour qu'une concentration C d'un médicament dans un liquide biologique ou un tissu atteigne la concentration C/2.

L'aire sous la courbe, ASC, ou AUC «Area under curve», correspond à l'intégrale de la concentration plasmatique sur un intervalle de temps défini

L'ASC s'exprime en masse (mg, g) x litre-1 x heure, est permet la mesure de la biodisponibilité d'un médicament.

Au sens de la présente invention, on entend par profil ayant une «fluctuation réduite», un profil ayant un swing $\Delta$ inférieur à 50% et pouvant être assimilée au plateau de concentrations que l'on obtiendrait après administration intraveineuse (IV) d'un opioïde.

Au sens de la présente invention, on entend également par profil ayant une «fluctuation réduite», un profil plasmatique maintenu au dessus de 60% ou de préférence au dessus de 75% de la valeur du $Css_{max}$ pendant au moins 10 heures.

BREVE DESCRIPTION DES FIGURES

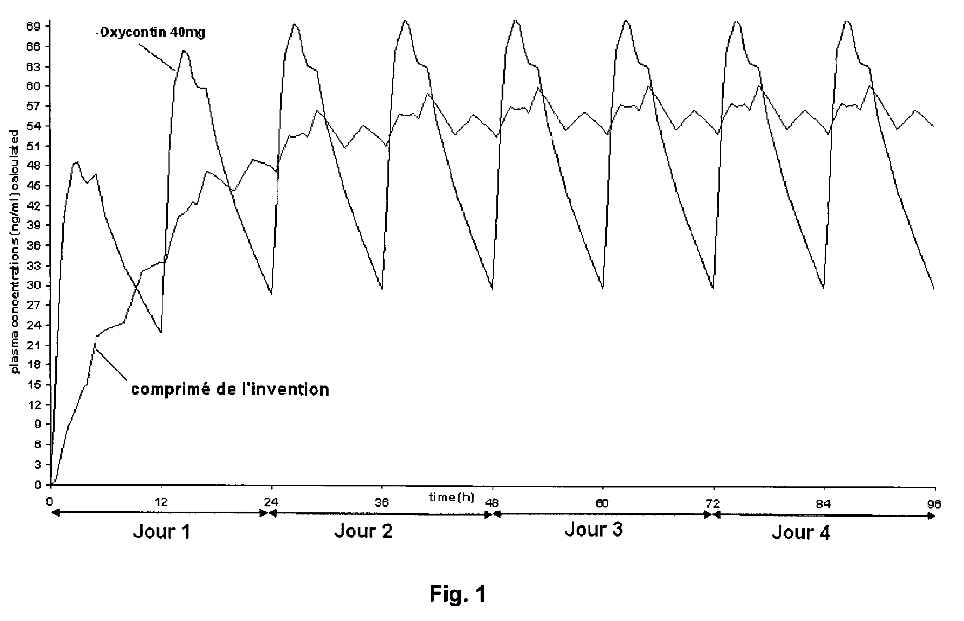

La figure 1 représente les profils plasmatiques d'oxycodone à l'état d'équilibre (steady-state) calculés par la méthode « Interpolation-Addition », des comprimés d'oxycodone dosés à 40 mg conformes à l'invention et des comprimés d'oxycodone du produit de référence Oxycontin® dosés à 40 mg.

Au sens de la présente invention, on entend par «profil pharmacocinétique calculé» par le modèle «interpolation-addition», le profil résultant de l'addition et la superposition toutes les $\tau$ heures du profil cinétique obtenu chez l'homme après administration unique du produit. La méthode de calcul est soit manuelle, soit automatisée par l'utilisation du logiciel Pharmacocinétique Win Non Lin@, plus particulièrement de l'outil «non parametric superposition» de ce logiciel.

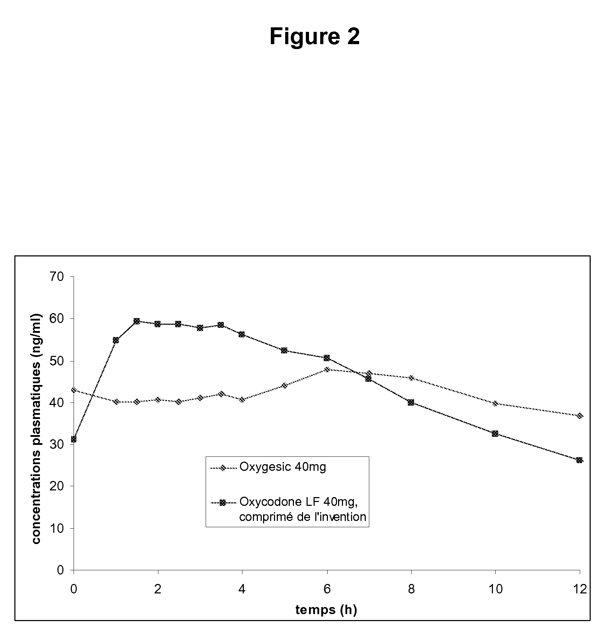

La figure 2 représente, à l'état d'équilibre (après 5.5 jours d'administration, c'est-à-dire 11 doses), les profils plasmatiques moyens observés après administrations répétées d'une de 40mg d'Oxycodone 2 fois par jour à des volontaires sains : comprimés d'oxycodone dosés à 40mg conformes à l'invention et comprimés d'oxycodone du produit de référence Oxygesic® dosés à 40mg, sur l'intervalle d'administration (12h) (en l'occurrence les 12 premières heures du jour 6)

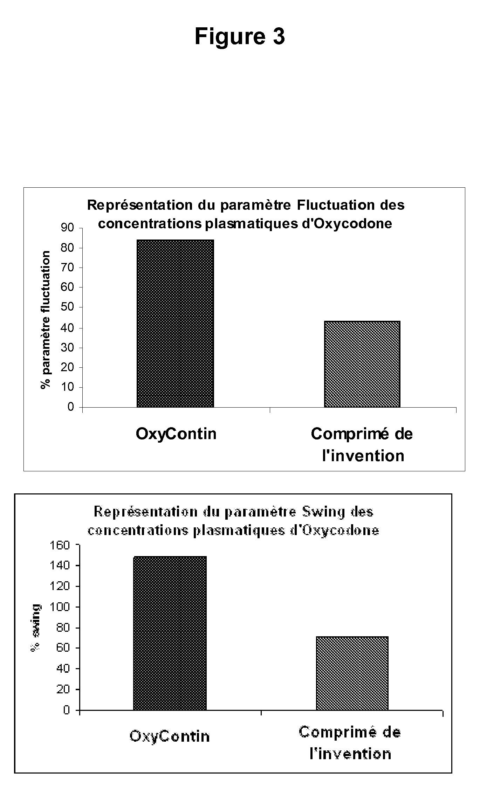

La figure 3 représente les paramètres « fluctuations et swing » observés (moyenne des paramètres individuels) après administration toutes les 12 heures à des volontaires sains pendant 5.5 jours (11 doses) des comprimés d'oxycodone dosés à 40 mg conformes à l'invention et des comprimés d'oxycodone du produit de référence Oxycontin® dosés à 40 mg.

DESCRIPTION DETAILLEE DE L'INVENTION

Il a été découvert dans le cadre de cette invention que des formulations pharmaceutiques préparées sous la forme d'un comprimé matriciel à libération prolongée peuvent être administrées à un humain, de façon répétée, deux fois par jour, de telle sorte que la concentration plasmatique en opioïde obtenue in vivo à l'état d'équilibre est maintenue, avec une fluctuation réduite, au dessus de 60% de la valeur du $Css_{max}$, de préférence au dessus de 75% de la valeur du $Css_{max}$ pendant au moins 10 heures, de préférence au moins 12 heures, c'est-à-dire pendant tout l'intervalle $\tau$ et encore plus de préférence de 12 heures jusqu'à 20 heures.

Par conséquent, l'invention concerne un comprimé matriciel à libération prolongée d'au moins un opioïde ou d'un de ses sels pharmaceutiquement acceptables, la matrice de compression dudit comprimé matriciel étant constituée d'au moins un excipient choisi dans le groupe comprenant les polymères retard pH indépendants et insolubles dans l'eau, les excipients minéraux et leurs mélanges tel que défini dans la revendication 1, pour son utilisation en tant que médicament administré par voie orale de façon répétée à raison de deux prises par jour espacées de 8 à 14 heures, de préférence 11 à 13 heures, de manière particulièrement préférée 12 heures.

L'invention concerne également l'utilisation d'un comprimé matriciel à libération prolongée d'au moins un opioïde ou d'un de ses sels pharmaceutiquement acceptables, la matrice de compression dudit comprimé matriciel étant constituée d'au moins un excipient choisi dans le groupe comprenant les polymères retard pH indépendants et insolubles dans l'eau, les excipients minéraux et leurs mélanges pour la fabrication d'un médicament administré par voie orale de façon répétée à raison de deux prises par jour espacées de 8 à 14 heures, de préférence 11 à 13 heures, de manière particulièrement préférée 12 heures.

Ledit médicament est destiné au traitement de la douleur.

Avantageusement, le comprimé selon l'invention permet de maintenir la concentration plasmatique en opioïde obtenue in vivo à l'état d'équilibre (steady-state) avec une fluctuation réduite au dessus de 60% de la valeur du Css$_{max}$, de préférence au dessus de 75% de la valeur du Css$_{max}$, pendant plus de 8 heures, de préférence pendant au moins 10 heures et de manière encore préférée au moins 12 heures. Un tel résultat n'a jusqu'à présent pas été rapporté avec les formulations de l'art antérieur du type granules ou du type comprimés à action immédiate ou à action retard, en une ou deux prises par jour.

L'utilisation des comprimés matriciels selon l'invention permet d'atteindre à l'état d'équilibre (au steady state), une pharmacocinétique proche de celle d'une perfusion à débit constant (la vitesse d'entrée équilibrant la vitesse d'élimination), avec un minimum de variations dans les concentrations plasmatique en opioïde. Il s'agit donc en diminuant les fluctuations plasmatiques, de diminuer les pics et vallées (« peak and trough »), et donc de limiter les épisodes de douleurs aigües (breakthrough pain).

La matrice à libération prolongée du comprimé matriciel utilisé selon l'invention est constituée d'au moins un excipient choisi dans le groupe comprenant les polymères retard pH indépendants et/ou insolubles dans l'eau, les excipients minéraux et leurs mélanges, tel que défini dans la revendication 1.

Avantageusement, l'utilisation des comprimés matriciels à libération prolongée de la présente invention permet d'obtenir à l'état d'équilibre après administration toutes les 10 à 14 heures, de préférence toutes les 12 heures des niveaux

Avantageusement, l'utilisation des comprimés matriciels à libération prolongée selon l'invention présente d'une part un paramètre «swing» inférieur, à 50%, de préférence inférieur à 25% et d'autre part un paramètre « fluctuation » inférieur à 50%, de préférence inférieur à 25% au steady state après administration répétée toutes les 10 à 14 heures, de préférence toutes les 12h.

Encore plus avantageusement, l'utilisation selon l'invention toutes les 10 à 14 heures, de préférence toutes les 12 heures des comprimés matriciels à libération prolongée de la présente invention permet d'observer des variations de concentrations au steady state (différence entre Css$_{max}$ et Css$_{min}$) réduites à moins de 30% de la valeur du C$_{max}$, de préférence réduites à moins de 20% de la valeur du C$_{max}$ et encore plus préférentiellement réduites jusqu'à environ 14%.

Encore plus avantageusement, l'utilisation selon l'invention des comprimés matriciels à libération prolongée de l'invention permet de diminuer les fluctuations plasmatiques, de diminuer les « pics et vallées » («peak and trough»), et donc de limiter les résurgences de la douleur liées à des diminutions de concentrations, voir de limiter les épisodes de douleurs aigües (breakthrough pain). Grâce aux valeurs de concentrations maximales réduites, l'utilisation selon la présente invention permet de diminuer certains effets secondaires tels que nausées, somnolence ou autres effets secondaires cognitifs.

L'utilisation des comprimés matriciels à libération prolongée de l'invention présente une dissolution et une libération d'opioïde de moins de 30% en 4 heures, moins de 80% en 16 heures, telle que mesurée conformément à la méthode de la palette tournante à 100 t/min selon la Pharmacopée US, dans un milieu de dissolution constitué de 900mL de milieu aqueux pH 6.8, de telle sorte que la C$_{max}$ en opioïde, obtenue in vivo, soit atteinte 8 à 14 heures et de préférence 10 à 12 heures après administration unique de la formulation.

Les comprimés conformes à l'invention permettent, une libération prolongée dans le temps du ou des principes actifs opioïdes contenus dans ladite matrice. Les comprimés conformes à l'invention permettent ainsi une libération du principe actif dans l'organisme dont la durée est supérieure à 8 heures, préférentiellement supérieure à 12 heures, encore plus préférentiellement supérieure à 20 heures.

La matrice de compression des comprimés conformes à l'invention représentera 50 à 98% en poids du poids total des comprimés, encore plus avantageusement, 85 et 95% en poids du poids total desdits comprimés.

Les polymères retards, insolubles dans l'eau et pH indépendants utilisables dans la constitution de la matrice des comprimés selon l'invention sont les mélanges de polymères tels que le mélange de cellulose microcristalline et d'acétate de polyvinyle/polyvinylpyrrolidone (80 :20) (vendu sous le nom de marque Kollidon®SR).

Les polymères retards, insolubles dans l'eau et pH indépendants conformes à la présente invention appartiennent au groupe comprenant les dérivés cellulosiques, le mélange de cellulose microcristalline et d' [acétate de polyvinyle/polyvinylpyrrolidone (80 :20) (vendu sous le nom de marque Kollidon®SR)].

Selon un mode de réalisation avantageux de l'invention, la matrice de compression est constituée d'un mélange (1:1) de cellulose microcristalline et du mélange [acétate de polyvinyle/polyvinylpyrrolidone en proportion 80:20 (vendus sous le nom de marque Kollidon® SR )]. Avantageusement ces deux polymères représentent chacun une proportion en poids entre 35 et 45% du poids total de ladite matrice de compression.

La matrice de compression peut avantageusement, outre les excipients de la matrice de compression contenir un ou plusieurs excipients destinés soit à favoriser le déroulement du processus de compression tels que des agents anti-collage comme la silice colloïdale, le talc, le stéarate de magnésium, le Polyéthylène Glycol (PEG) ou le stéarate de calcium, soit à améliorer la cohésion des comprimés lors de la compression, tels que les agents liants utilisés classiquement dans cette fonction, en particulier les amidons, les dérivés cellulosiques, soit des agents de charge, soit des lubrifiants, soit des plastifiants, soit des agents de remplissage, soit des édulcorants soit des colorants. Quand ils sont présents, ces excipients sont utilisés de façon classique à raison de 0,1 à 10 % en poids du poids total de la matrice de compression, préférentiellement de 0,5 à 5 % en poids.

Selon un mode particulier de l'invention, les comprimés sont pelliculés à l'aide d'un enrobage externe. On pourra utiliser à cette fin, différents grades d'éthylcellulose ou de polymères méthacryliques bien connus de l'homme de l'art.

Le ou les excipients utilisés pour l'enrobage sont appliqués de façon connue de l'homme du métier en quantité nécessaire pour obtenir la ou les fonctions recherchées.

Ces excipients peuvent être appliqués à la surface du comprimé de façon classique par pulvérisation d'une solution ou suspension de l'agent d'enrobage dans un solvant, en turbine perforée ou en lit fluidisé par exemple.

Lorsque le polymère d'enrobage du comprimé est un polymère retard, les comprimés enrobés conformes à l'invention peuvent avantageusement subir une phase de maturation dudit polymère d'enrobage afin de garantir sa stabilité physique et chimique. Cette étape est réalisée dans des conditions de température maîtrisée, inférieure à la température de fusion du principe actif pendant un temps contrôlé qui dépend du polymère d'enrobage et qui peut être compris entre 1 minute et plusieurs mois, pour un taux d'humidité relatif de 50 à 99%. Cette étape peut être réalisée en étuve ou en turbine.

L'enrobage peut-être réalisé à partir d'une dispersion aqueuse d'éthylcellulose (Aquacoat® ECD-30, FMC), avantageusement dans une proportion d'éthylcellulose représentant 2 à 5% en poids du poids total des comprimés enrobés.

Les opioïdes utilisés dans le cadre de l'invention sont préférentiellement des dérivés et/ou des alcaloïdes de l'opium, naturels ou de synthèse telles que la codéine, la narcéine, la noscapine et leurs sels. Les actifs utilisables selon l'invention appartiennent en outre au groupe comprenant la morphine, ses dérivés et leurs sels et en particulier les morphinènes tels que la pholcodine, la nalorphine, la codéine, la dihydrocodéine, l' hydromorphone, et les morphinanes tels que la buprenorphine, le butorphanol, le dextromethorphane, la nalbufine, la naltrexone, le naloxone, le nalmefene, l'hydrocodone, l'oxymorphone et l'oxycodone et d'une manière générale tous les analogues de la morphine et tous les analgésiques morphiniques, tels que le fentanyle, le tramadol, l'apomorphine et l'étorphine.

Le ou les principe (s) actif (s) constituant les comprimés selon l'invention peu(ven)t représenter entre 5 et 70% en poids du poids total du comprimé. Avantageusement le ou les actifs représente(nt) 10 à 50% en poids du poids total du comprimé. Le (s) principe (s) actif (s) peu (ven) t être directement introduit (s) dans le mélange pour compression, monté (s) sur supports (obtention de microgranules) ou granulé (s) par voie humide ou sèche (obtention de granules).

Lorsque le ou les principe (s) actif (s) sont présents sous la forme de microgranules, ces microgranules peuvent être obtenus de façon classique par dépôt (montage) du ou des actifs à la surface de supports pharmaceutiquement neutres, tels que des microbilles préfabriquées à base de cellulose ou d'un mélange de sucre et d'amidon et vendues sous le terme "neutral core" ou " sugar sphères" ou encore des granulés d'autres excipients, comme le lactose par exemple. Le procédé de dépôt (montage) de l'actif est réalisé de façon classique et connue de l'homme du métier et peut varier en fonction de la nature, de la quantité et de la fragilité du ou des principes actifs à déposer. Ainsi, le dépôt (montage) peut s'effectuer par pulvérisation d'une solution ou suspension du ou des principes actifs à la surface du support neutre ou la pulvérisation du ou des actifs en poudre à la surface du support préalablement humidifié à l'aide d'une solution d'agent liant.

Les granules de principe (s) actif (s) peuvent également être obtenus par granulation par voie sèche ou par voie humide du ou des principes actifs d'intérêt, généralement en présence d'au moins un agent liant et d'un liquide de mouillage le cas échéant, selon des techniques, là encore, bien connues de l'homme de l'art. Les granules ainsi obtenus sont mélangés avec les excipients de la matrice de compression puis le mélange est comprimé.

Avantageusement, les granules comportant le ou les principes actifs d'intérêt ont un diamètre permettant l'obtention d'un bon rendement de compression c'est à dire généralement compris entre 100 et 600 μm.

Selon un autre mode de réalisation de l'invention, et lorsque sa granulométrie le permet, le principe actif est mélangé directement avec les excipients constituant la matrice de compression puis le mélange est directement comprimé.

Enfin, un autre mode possible de réalisation de l'invention consiste à mélanger le principe actif avec le ou les excipients de la matrice de compression, puis à granuler ce mélange par voie sèche ou humide afin d'obtenir des granules directement compressibles.

Avantageusement, la surface totale du comprimé est inférieure à 150 mm². La présente invention est donc adaptée à la fabrication tant de comprimés faiblement dosés en actif que de comprimés fortement dosés.

L'actif peut-être mélangé directement dans la matrice de compression ou être mélangé sous forme de granules ou de microgranules préparés au préalable. Cette étape de granulation améliore l'uniformité de teneur des comprimés fabriqués. Elle est réalisée de façon préférentielle en voie humide (aqueuse ou organique) pour les granules ou par dépôt (montage) du principe actif en solution ou en suspension sur supports neutres pour les microgranules.

La compression est réalisée sur machine à comprimer rotative avec station de pré-compression. Les paramètres de compression doivent être choisis pour permettre de générer des comprimés de dureté adaptée à la présente invention.

Ainsi, selon l'invention, un mode de réalisation préféré des comprimés matriciels à libération prolongée comprend la formulation suivante:

| Ingrédients | Pourcentage (%) |
|---|---|
| Granules | |
| Opioïde | 10-50 |
| Agent liant | 0,1-10 |
| Acétate de polyvinyle/ polyvinylpyrrolidone (80:20) | 35-45 |
| Cellulose microcristalline | 35-45 |
| Enrobage | |
| Polymère retard | 2-5 |
| Lubrifiants | 0,1-1 |
| Plastifiants | 0,1-2 |

De manière typique, les formulations de la présente invention comprennent un dosage en opioïde compris de 1 à 400 mg, par exemple, 1, 2.5, 5, 10, 15, 25, 40, 50, 60, 80, 120, 160, 200, 300 et 400 mg.

Les exemples qui suivent ont pour but d'illustrer l'invention.

EXEMPLES

EXEMPLE 1

Fabrication de comprimés à base de granules obtenus par granulation d'oxycodone HCl et d'HPMC et d'une matrice de compression constituée d'un mélange (1:1) de deux excipients [cellulose microcristalline et (PVA/povidone 80:20)].

Préparation des comprimés

1.1. Préparation des granules d'oxycodone

Les granules sont obtenus par granulation humide du principe actif (oxycodone HCl ; Mc Farlan Smith, Angleterre) et de hydroxypropylméthylcellulose (HPMC grade Pharmacoat® 606, Brenntag) qui joue le rôle de liant. La granulation est réalisée en lit fluidisé (GCPG-I, Wurster, Glatt, Allemagne) par projection en mode bottom-spray d'une solution du liant (HPMC) sur le principe actif sous forme de poudre.

L'oxycodone est introduite dans la cuve du lit fluidisé et mise en sustentation. La solution liante est pulvérisée sur la poudre qui s'agglomère pour former les granules. L'eau est éliminée progressivement par évaporation puis à l'issue d'une étape finale de séchage. L'étape finale de séchage en étuve (16 heures à 60°C est réalisée pour obtenir une teneur en eau finale acceptable (inférieure à 6%). Les proportions d'HPMC et d'oxycodone figurent dans le tableau 1.

Tableau 1

| Ingrédients | Pourcentage [%] | Masse (g/lot) |
|---|---|---|
| Oxycodone HCl | 93.54 | 590.5 |
| HPMC (Pharmacoat® 606) | 6.46 | 40.8 |
| Eau purifiée | - | 483.9 |
| Total (sec) | 100.0 | 631.3 |

1.2. Préparation de la matrice de compression

Un pré-mélange de cellulose microcristalline (Avicel(® PH102, FMC) et silice précipitée (Syloïd®244, Keyser & Mc Kay) est réalisé en mélangeur cubique (AR401, Erweka) pendant 2 min à 40 rpm. Le mélange polyvinylacétate/povidone (80:20) (Kollidon® SR, BASF) et les granules d'oxycodone préparés comme décrit à l'étape 1.1 sont ajoutés au pré-mélange et l'homogénéisation est réalisée en mélangeur cubique pendant 15 minutes à 40 rpm. Enfin le lubrifiant (stéarate de magnésium, Quimdis) destiné à limiter le collage et les frictions en compression est ajouté au mélange précédent selon les paramètres de mélange : 5 minutes à 40 rpm.

La quantité de granules d'oxycodone utilisée est déterminée de manière à fabriquer des comprimés dosés à 40 mg d' oxycodone.

Les proportions de chacun des excipients sont récapitulées dans le tableau 2.

Tableau 2

| Ingredients | Pourcentage [%] | Masse (mg/comprimé) |
|---|---|---|
| Comprimés | 95.96 | 227.00 |
| HPMC (603) | 2.88 | 6.81 |
| Simethicone (poids sec) | 0.01 | 0.02 |
| Talc | 0.86 | 2.03 |
| Syloïd® 244 | 0.29 | 0.69 |
| Eau purifiée** | N/A | N/A |
| Total (sec) | 100.00 | 234.5 |

1.3. Compression

L'étape de compression du mélange final obtenu à l'étape précédente est réalisée sur une presse à comprimer (PR-12), Sviac) sous une force de compression de 35 kN avec des poinçons oblongs de 11 mm x 5 mm. La compression s'effectue de manière conventionnelle sans que ni le mélange pour compression, ni les outils de compression ne soient soumis à une étape de chauffage avant ou pendant l'étape de compression proprement dite.

1.4 Enrobage

1.4.1 Sous-enrobage

Préalablement à l'enrobage par le polymère proprement dit, une étape de sous-enrobage est réalisée sur les comprimés.

Cette sous-couche est destinée à améliorer l'état de surface des comprimés. Elle est constituée d'un mélange d'HPMC (Pharmacoat® 603), d'un agent anti-mousse (Siméthicone, Dow Corning), d'un lubrifiant (talc micronisé, Luzenac (Univar) et d'un agent anti-statique (Syloid® 244, Keyser & McKay) de telle sorte que l'HPMC représente un gain en poids de 3% par rapport au poids total des comprimés nus. Les proportions de chacun des excipients figurent dans le tableau 3.

Ce sous-enrobage est réalisé de manière classique en turbine perforée (Trislot).

1.4.2. Enrobage

L'enrobage proprement dit des comprimés préalablement sous-enrobés est également réalisé en turbine perforée (Trislot).

L'enrobage est réalisé à partir d'une dispersion aqueuse d'éthylcellulose (Aquacoat® ECD-30, FMC) avec une proportion d'éthylcellulose représentant 2,87% en poids du poids total des comprimés enrobés. La proportion des différents excipients figure dans le tableau 3. Là encore, aucune étape spécifique de chauffage des comprimés n'est réalisée avant ou pendant l'application du sous-enrobage ou de l'enrobage proprement dit.

Tableau 3

| Ingrédients | Pourcentage [%] |
|---|---|
| Comprimés | 95.75 |
| Aquacoat® ECD-30 (sec) | 2.87 |
| Dibutyl sébacate | 0.69 |
| Talc | 0.52 |
| Syloïd® 244 | 0.17 |
| Eau purifiée** | N/A |
| Total (sec) | 100.00 |
| **Note: l'eau est éliminée pendant le procédé; N/A: Non Applicable | |

Etude Pharmacocinétique

1. Etude de Pharmacocinétique chez le volontaire sain

Les comprimés dosés à 40 mg fabriqués sont également testés in vivo afin de déterminer le profil plasmatique de l'oxycodone chez des volontaires sains auxquels on a administré une dose desdits comprimés.

Une étude pharmacocinétique est réalisée sur 12 volontaires sains mâles et femelles à jeun séparés en deux demi-groupes.

Chaque demi-groupe reçoit successivement les deux traitements (comprimés de l'invention et produit de référence) après une période intermédiaire sans administration («wash-out »).

Le produit de référence utilisé dans cette étude est l'Oxycontin®, comprimé d'oxycodone à libération prolongée administrable à raison de deux prises par jour, également dosé à 40 mg.

Les concentrations plasmatiques (exprimés en ng/ml) pour la mesure de l'évolution des concentrations sanguines d'oxycodone dans le sang ont été déterminés au moyen de prélèvements sanguins aux temps suivants : avant administration (t=0), puis à 0.5, 1, 1.5, 2, 2.5, 3, 3.5, 4, 5, 6, 8, 10, 12, 14, 16, 20, 24, 30, 36 et 48h après administration du produit (comprimé de l'invention ou produit de référence selon la randomisation).

Les concentrations plasmatiques d'oxycodone ont été mesurées après administration unique d'une dose orale chez 12 sujets sains. Les paramètres pharmacocinétiques résultants sont résumés dans le tableau 4.

Tableau 4

| Paramètres | Test (invention) (n=12) | | Reference Oxycontin® (n=12) | |
|---|---|---|---|---|
| | Moyenne | CV% | Moyenne | CV% |
| $C_{max}$ (ng/mL) | 34.412 | 20 | 53.129 | 25.0 |
| $T_{max}$ (heures) | 10.0 | 16.6 | 3.00 | 34.3 |
| AUCt (ng h/ mL) | 667.109 | 16.9 | 611.848 | 21.9 |
| $AUC_{\infty}$ (ng h/ mL) | 679.846 | 17.1 | 614.960 | 21.7 |
| $AUC_{t/\infty}$ (%) | 98.17 | 1.7 | 99.48 | 0.3 |
| $K_{el}$ (hours$^{-1}$) | 0.1154 | 24.0 | 0.1561 | 16.4 |

(suite)

| Paramètres | Test (invention) (n=12) | | Reference Oxycontin® (n=12) | |
|---|---|---|---|---|
| | Moyenne | CV% | Moyenne | CV% |
| $T_{1/2\ el}$ (hours) | 6.39 | 28.0 | 4.56 | 17.2 |
| Note : Pour les valeurs de $T_{max}$, c'est médiane qui est indiquée ; CV : Coefficient de variation en % ; $K_{el}$ : constante d'élimination ; $T_{1/2\ el}$: demi-vie d'élimination | | | | |

2. Etude in silico : simulation de la pharmacocinétique à l'état d'équilibre par « interpolation-addition » à partir des résultats de la dose unique.

On compare la biodisponibilité calculée du comprimé de l'invention à celle d'une formulation d'oxycodone de référence (OxyContin® dosé à 40 mg), après calcul des profils au steady state de chacun des volontaires à partir de leur propre profil à dose unique (simulation d'une dose répétée de 4 jours chez 12 volontaires sains). Le profil moyen, les paramètres moyens sont donc la moyenne des profils individuels et des paramètres individuels. Ainsi, la variabilité interindividuelle est prise en compte dans le calcul.

Les paramètres pharmacocinétiques calculés à l'état d'équilibre de l'oxycodone sont rassemblés dans le tableau 5 qui suit :

Tableau 5

| | OxyContin® 40mg à l'équilibre (calculé) | | Oxycodone Test (invention) 40mg à l'équilibre (calculé) | |
|---|---|---|---|---|
| | Paramètre moyen | Min-Max | Paramètre moyen | Min - Max |
| Cssmax (ng/ml) | 74.25 | 62-121 | 62.7 | 48 - 79.5 |
| Cssmin (ng/ml) | 29.3 | 21.9-41.7 | 50.7 | 36.7 - 65.5 |
| Swing (%) | 156.6 | 105.7 - 218.7 | 24.1 | 12.8 - 43.7 |
| Fluctuation (%) | 87.9 | 66.6 - 117.2 | 21.4 | 12.1 - 37 |
| Cav (ng/ml) | 51.2 | 39.4 - 79.2 | 56.5 | 43.4 - 69.6 |
| AUCsst (ng/ml.h) | 614.6 | 472.4 - 950.4 | 678.4 | 520.9 - 835.7 |
| Cssmax-Cssmin (ng/ml) | 60.5 | 54.3 - 65.5 | 19.1 | 14.4 - 30.4 |

Les paramètres « fluctuation » et « swing » de la formulation selon l'invention présentent des valeurs arrondies respectives de 21 et 24%.

3. Bioéquivalence in silico : Bioéquivalence effectuée à partir des paramètres individuels calculés

| | Ratio | 90% Confidence interval |
|---|---|---|
| AUCt | 113.3 | 105.3 - 121.9 |
| Swing | 14.34 | 11.84 - 17.35 |
| Fluctuation | 22.53 | 19.08 - 26.6 |

Les paramètres « fluctuation » et « swing » présentent un ratio par rapport à la référence respectivement de 14.3% et 22.5%, soit une réduction de 85% et 77%.

EXEMPLE 2

1. Fabrication de comprimés pelliculés à l'aide d'un enrobage externe d'Aquacoat® ECD-30 (Ethylcellulose) à base de granules obtenus par granulation d'oxycodone HCl et de HPMC et d'une matrice de compression constituée d'un mélange (1 :1) de deux excipients [cellulose microcristalline et (PVA/povidone 80:20)]1

Préparation des comprimés

1.1. Préparation des granules d'oxycodone

Les granules sont obtenus par granulation humide du principe actif (oxycodone HCl ; Mc Farlan Smith, Angleterre) et de hydroxypropylméthylcellulose (HPMC grade Pharmacoat® 606, Brenntag) qui joue le rôle de liant. La granulation est réalisée en lit fluidisé (GCPG-I, Wurster, Glatt, Allemagne) par projection en mode bottom-spray d'une solution du liant (HPMC) sur le principe actif sous forme de poudre.

L'oxycodone est introduite dans la cuve du lit fluidisé et mise en sustentation. La solution liante est pulvérisée sur la poudre qui s'agglomère pour former les granules. L'eau est éliminée progressivement par évaporation puis à l'issue d'une étape finale de séchage. L'étape finale de séchage en étuve (16 heures à 60°C est réalisée pour obtenir une teneur en eau finale acceptable (inférieure à 6%). Les proportions d'HPMC et d'oxycodone figurent dans le tableau 6.

Tableau 6

| Ingredients | Pourcentage[%] |
|---|---|
| Oxycodone HCl | 95.9 |
| HPMC (Pharmacoat® 606) | 4.1 |
| Eau purifiée | - |
| Total | 100.0 |

1.2. Préparation de la matrice de compression

Un pré-mélange de cellulose microcristalline (Avicel(® PH102, FMC) et silice précipitée (Syloïd®244, Keyser & Mc Kay) est réalisé en mélangeur cubique (AR401, Erweka) pendant 2 min à 40 rpm. Le mélange polyvinylacétate/povidone (80:20) (Kollidon® SR, BASF) et les granules d' oxycodone préparés comme décrit à l'étape 1.1 sont ajoutés au pré-mélange et l'homogénéisation est réalisée en mélangeur cubique pendant 15 minutes à 40 rpm. Enfin le lubrifiant (stéarate de magnésium, Quimdis) destiné à limiter le collage et les frictions en compression est ajouté au mélange précédent selon les paramètres de mélange : 5 minutes à 40 rpm.

La quantité de granules d'oxycodone utilisés est déterminée de manière à fabriquer des comprimés dosés à 40 mg d' oxycodone.

Les proportions de chacun des excipients sont récapitulées dans le tableau 7.

Tableau 7

| Ingredients | Pourcentage [%] | Masse (mg/comprimé) |
|---|---|---|
| Granules d'Oxycodone | 19.47 | 43.80 |
| Kollidon® SR | 39.92 | 89.81 |
| Avicel® PH102 | 39.92 | 89.81 |
| Syloïd® 244 | 0.20 | 0.46 |
| Stéarate de Magnesium | 0.50 | 1.13 |
| Total | 100.00 | 225.00 |

1.3. Compression

L'étape de compression du mélange final obtenu à l'étape précédente est réalisée sur une presse à comprimer (PR-12), Sviac) sous une force de compression de 35 kN avec des poinçons oblongs de 11 mm x 5 mm. La compression s'effectue de manière conventionnelle sans que ni le mélange pour compression, ni les outils de compression ne soient soumis à une étape de chauffage avant ou pendant l'étape de compression proprement dite.

### 1.4. Enrobage

L'enrobage proprement dit des comprimés est réalisé en turbine perforée (Trislot ou Glatt).

L'enrobage est réalisé à partir d'une dispersion aqueuse d'éthylcellulose (Aquacoat® ECD-30, FMC) avec une proportion d'éthylcellulose représentant 2,87% en poids du poids total des comprimés enrobés. La proportion des différents excipients figure dans le tableau 8.

Une étape de chauffage des comprimés est réalisée pendant l'application de l'enrobage dit.

Tableau 8

| Ingredients | Pourcentage [%] |
|---|---|
| Comprimés | 95.75 |
| Aquacoat® ECD-30 (sec) | 2.87 |
| Dibutyl sebacate | 0.69 |
| Talc | 0.52 |
| Syloïd® 244 | 0.17 |
| Eau purifiée** | N/A |
| Total (sec) | 100.00 |
| **Note: l'eau est éliminée pendant le procédé; N/A: Non Applicable | |

### 2. Etude de Pharmacocinétique en doses répétées chez le volontaire sain: caractérisation du profil pharmacocinétique du comprimé de l'invention à l'état d'équilibre.

Une étude pharmacocinétique est réalisée sur 30 volontaires sains mâles et femelles à jeun séparés en deux demi-groupes.

Chaque demi-groupe reçoit successivement les deux traitements (comprimés de l'invention et produit de référence chacun administrés 2 fois par jour pendant 5.5 jours (11 traitements par période)) après une période intermédiaire sans administration («wash-out »).

Le produit de référence utilisé dans cette étude est l'Oxygesic®, comprimé d' oxycodone à libération prolongée administrable à raison de deux prises par jour, également dosé à 40 mg.

Les concentrations plasmatiques (exprimés en ng/ml) pour la mesure de l'évolution des concentrations sanguines d'oxycodone dans le sang ont été déterminés au moyen de prélèvements sanguins aux temps suivants :

- avant les administrations des jours 4 et 5 afin de vérifier l'atteinte de l'état d'équilibre,
- puis 5 minutes avant la dernière administration (jour 6), et 1, 1.5, 2, 2.5, 3, 3.5, 4, 5, 6, 7, 8, 10, 12, 14, 16, 20, 24, 36 et 48h après cette dernière administration du produit (comprimé de l'invention ou produit de référence selon la randomisation).

Les concentrations plasmatiques d'oxycodone ont donc été mesurées après administrations répétées toutes les 12 heures d'une dose orale de 40mg chez 30 sujets sains.

Les paramètres pharmacocinétiques observés à l'état d'équilibre de l'oxycodone sont rassemblés dans le tableau 9 qui suit :

Tableau 9

| | Oxygesic® 40mg à l'équilibre | | Oxycodone Test (invention) à l'équilibre | |
|---|---|---|---|---|
| | Paramètre moyen | CV (%) | Paramètre moyen | CV (%) |
| Cssmax (ng/ml) | 63.792 | 20.7 | 51.719 | 25.3 |
| Cssmin (ng/ml) | 26.299 | 27.2 | 36.890 | 40.2 |

(suite)

|  | Oxygesic® 40mg à l'équilibre | | Oxycodone Test (invention) à l'équilibre | |
|---|---|---|---|---|
|  | Paramètre moyen | CV (%) | Paramètre moyen | CV (%) |
| Swing (%) | 148.13 | 22.9 | 70.38 | 113 |
| Fluctuation (%) | 83.86 | 16.8 | 42.86 | 57.6 |
| Cav (ng/ml) | 45.411 | 23.0 | 42.365 | 27.0 |
| AUCSST (ng/ml.h) | 544.937 | 23.0 | 508.381 | 27.0 |
| Cssmax-Cssmin (ng/ml) | 17.65 | 8.66 | 37.49 | 8.37 |

Les paramètres « fluctuation » et « swing » de la formulation selon l'invention présentent des valeurs arrondies respectives de 43 et 70%.

Les paramètres « fluctuation » et « swing » présentent un ratio par rapport à la référence respectivement de 44 % et 33%, soit une réduction de 56% et 67%.

Conclusion

Sur le plan pharmacocinétique, les caractéristiques de la présente invention ont permis de mettre en évidence les avantages suivants :

- Réduction des valeurs de concentrations maximales à l'état d'équilibre, tout en conservant les mêmes AUC (même biodisponibilité)
- Diminution des fluctuations au steady state (paramètres fluctuation et swing, différence entre Cssmax et Cssmin)
- Maintien des concentrations au dessus de la valeur 75% du Cssmax pendant un intervalle de temps augmenté.

Sur le plan clinique, les caractéristiques de libération des formes galéniques d'une substance pharmaceutiquement active ont permis de découvrir les avantages suivants :

- maintien de concentrations plasmatiques efficaces et quasi constantes à l'état d'équilibre (pratiquement sans fluctuation ni swing) lors d'un traitement chronique, avec des prises répétées de la forme galénique de la présente invention toutes les 12 heures.
- Maintien des concentrations plasmatiques à l'état d'équilibre à un niveau réduit, limitant ainsi l'apparition d'effets secondaires tels que nausées, somnolence ou autres effets secondaires cognitifs.
- Limitation des « pics et vallées » ou « peak and through », avec limitation de l'apparition de douleurs résurgentes liées aux concentrations plasmatiques, et limitation des breakthrough pain

**Revendications**

1. Comprimé matriciel à libération prolongée d'au moins un opioïde ou d'un de ses sels pharmaceutiquement acceptables, comprenant une matrice de compression qui représente 50 à 98% en poids du poids total dudit comprimé et est constituée d'un mélange de cellulose microcristalline et d'acétate de polyvinyle/ polyvinylpyrrolidone (80:20), pour son utilisation pour limiter les épisodes de douleurs aigües par son administration par voie orale de façon répétée à raison de deux prises par jour espacées de 8 à 14 heures, de préférence 10 à 13 heures.

2. Comprimé matriciel pour son utilisation selon la revendication 1, enrobé par un polymère à effet retard, de préférence l'éthylcellulose.

3. Comprimé matriciel pour son utilisation selon la revendication 1 ou 2, maintenant la concentration plasmatique en opioïde obtenue in vivo à l'état d'équilibre (steady-state) avec une fluctuation réduite au dessus de 60% de la valeur du $Css_{max}$, de préférence au dessus de 75% de la valeur du $Css_{max}$ pendant au moins 10 heures et de préférence au moins 12 heures.

4. Comprimé matriciel pour son utilisation selon l'une quelconque des revendications 1 à 3 **caractérisé par** un paramètre «swing» inférieur à 50%, de préférence inférieur à 25%.

**5.** Comprimé matriciel pour son utilisation selon l'une quelconque des revendications 1 à 4, **caractérisé par** un paramètre «fluctuation» inférieur à 50%, de préférence inférieur à 25%.

**6.** Comprimé matriciel pour son utilisation selon l'une quelconque des revendications 1 à 5, **caractérisé par** une diminution des fluctuations plasmatiques et des «pics et vallées», par une limitation des résurgences de la douleur liées à des diminutions de concentrations et par une limitation des épisodes de douleurs aigües.

**7.** Comprimé matriciel pour son utilisation selon l'une quelconque des revendications 1 à 6, **caractérisé par** une diminution de certains effets secondaires tels que nausées, somnolence ou autres effets secondaires cognitifs.

**8.** Comprimé matriciel pour son utilisation selon l'une quelconque des revendications 1 à 7, **caractérisé en ce que** l'opioïde est choisi dans le groupe comprenant la codéine, la narcéine, la noscapine, la morphine, la pholcodine, la nalorphine, la dihydrocodéine, l'hydromorphone, la buprenorphine, le butorphanol, le dextromethorphane, la nalbufine, la naltrexone, le naloxone, le nalmefene, l'hydrocodone, l'oxymorphone et l'oxycodone, le fentanyle, le tramadol, l'apomorphine et l'étorphine ou un de leurs sels pharmaceutiquement acceptables.

**9.** Comprimé matriciel pour son utilisation selon la revendication 8, **caractérisé en ce que** l'opioïde est le chlorhydrate d'oxycodone.

## Patentansprüche

**1.** Matrizentablette mit verlängerter Freisetzung wenigstens eines Opioids oder eines seiner pharmazeutisch akzeptablen Salze, umfassend eine Kompressions-matrix, die 50 bis 98 Gew.-% des Gesamtgewichts der genannten Tablette darstellt und aus einer Mischung aus mikrokristalliner Zellulose und Polyvinyl- / Polyvinylpyrrolidon-Acetat (80:20) gebildet ist, für ihre Verwendung zur Begrenzung von akuten Schmerzepisoden durch ihre Verabreichung auf oralem Weg auf wiederholte Weise in zwei Gaben pro Tag, die um 8 bis 14 Stunden, bevorzugt 10 bis 13 Stunden, beabstandet sind.

**2.** Matrizentablette für ihre Verwendung gemäß Anspruch 1, ummantelt mit einem Polymer mit Verzögerungswirkung, bevorzugt Ethylzellulose.

**3.** Matrizentablette für ihre Verwendung gemäß Anspruch 1 oder 2, die die in vivo erhaltene plasmatische Opioidkonzentration im Gleichgewichtszustand (steady-state) mit einer reduzierten Fluktuation während mindestens 10 Stunden, bevorzugt wenigstens 12 Stunden, oberhalb von 60 % des Wertes des $Css_{max}$, bevorzugt oberhalb von 75 % des Wertes des $Css_{max}$ hält.

**4.** Matrizentablette für ihre Verwendung gemäß irgendeinem der Ansprüche 1 bis 3, **gekennzeichnet durch** einen "Swing"-Parameter von weniger als 50 %, bevorzugt weniger als 25 %.

**5.** Matrizentablette für ihre Verwendung gemäß irgendeinem der Ansprüche 1 bis 4, **gekennzeichnet durch** einen "Fluktuations"-Parameter von weniger als 50 %, bevorzugt weniger als 25 %.

**6.** Matrizentablette für ihre Verwendung gemäß irgendeinem der Ansprüche 1 bis 5, **gekennzeichnet durch** eine Abnahme der plasmatischen Fluktuationen und der "Spitzen und Tiefen" **durch** eine Begrenzung des Wiederauftretens des Schmerzes in Verbindung mit Abnahmen der Konzentrationen und **durch** eine Begrenzung der Episoden von akuten Schmerzen.

**7.** Matrizentablette für ihre Verwendung gemäß irgendeinem der Ansprüche 1 bis 6, **gekennzeichnet durch** eine Abnahme von bestimmten Nebenwirkungen, wie z. B. Übelkeit, Schläfrigkeit oder sonstige kognitive Nebenwirkungen.

**8.** Matrizentablette für ihre Verwendung gemäß irgendeinem der Ansprüche 1 bis 7, **dadurch gekennzeichnet, dass** das Opioid aus der Gruppe ausgewählt ist, umfassend Kodein, Narcein, Noscapin, Morphin, Pholcodin, Nalorphin, Dihydrokodein, Hydromorphin, Buprenorphin, Butorphanol, Dextromethorphan, Nalbufin, Naltrexon, Naloxon, Nalmefen, Hydrocodon, Oxymorphon und Oxycodon, Fentanyl, Tramadol, Apomorphin und Etorphin oder eines ihrer pharmazeutisch akzeptablen Salze.

**9.** Matrizentablette für ihre Verwendung gemäß Anspruch 8, **dadurch gekennzeichnet, dass** das Opioid Oxycodon-Chlorhydrat ist.

**Claims**

**1.** Sustained-release matrix-type tablet releasing at least one opioid or one of its pharmaceutically acceptable salts, comprising a compression matrix which represents 50 to 98% by weight of the total weight of said tablet and comprised of a mixture of microcrystalline cellulose and of polyvinyl acetate/polyvinylpyrrolidone (80:20), for its use for limiting acute pain episodes through its administration orally in a repeated manner twice-a-day each administration being spaced apart by 8 to 14 hours, preferably 10 to 13 hours.

**2.** Matrix-type tablet for its use according to claim 1, coated with a sustained-release polymer, preferably ethylcellulose.

**3.** Matrix-type tablet for its use according to claim 1 or 2, which maintains the steady-state opioid plasma concentration obtained *in vivo* with a reduced fluctuation at above 60 % of the $Css_{max}$ value, preferably above 75 % of the $Css_{max}$ value for at least 10 hours and preferably at least 12 hours.

**4.** Matrix-type tablet for its use according to any of claims 1 to 3, **characterized by** a "swing" parameter of less than 50 %, preferably less than 25 %.

**5.** Matrix-type tablet according to any of claims 1 to 4, **characterized by** a "fluctuation" parameter of less than 50 %, preferably less than 25 %.

**6.** Matrix-type tablet for its use according to any of claims 1 to 5, **characterized by** a reduction in plasma fluctuations and in "peaks and troughs", by limitation of resurgent pain due to lowered concentrations, and by limitation of episodes of acute pain.

**7.** Matrix-type tablet for its use according to any of claims 1 to 6, **characterized by** a reduction in some side effects such as nausea, drowsiness or other cognitive side effects.

**8.** Matrix-type tablet for its use according to any of claims 1 to 7, **characterized in that** the opioid is chosen from the group comprising codeine, narceine, noscapine, morphine, pholcodine, nalorphine, dihydrocodeine, hydromorphone, buprenorphine, butorphanol, dextromethorphane, nalbufine, naltrexone, naloxone, nalmefene, hydrocodone, oxymorphone and oxycodone, fentanyl, tramadol, apomorphine and etorphine, or a pharmaceutically acceptable salt thereof.

**9.** Matrix-type tablet for its use according to claim 8, **characterized in that** the opioid is oxycodone hydrochloride.

Fig. 1

# Figure 2

# Figure 3

**Représentation du paramètre Fluctuation des concentrations plasmatiques d'Oxycodone**

**Représentation du paramètre Swing des concentrations plasmatiques d'Oxycodone**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2004084868 A
- EP 0609961 A
- WO 9600066 A
- US 4861598 A
- US 4970075 A
- WO 2007099154 A
- WO 20070099152 A
- WO 2007099152 A